# EUROPEAN PATENT APPLICATION

(11) **EP 3 826 030 A1**
(43) Date of publication of application: **26.05.2021**
(21) Application number: 20209860.4
(22) Date of filing: 25.11.2020
(51) Int. Cl.: G16H 40/20, G06Q 10/06, G06Q 50/00, G06Q 50/28, G16H 40/63, G05B 19/418, G05D 1/00, G05D 1/02, G06Q 10/08, A61L 2/00

(54) **MANAGEMENT SYSTEM, MANAGEMENT METHOD, PROGRAM, AND AUTONOMOUS MOVING APPARATUS**

(30) Priority: 25.11.2019 JP 2019212422
(71) Applicant: Toyota Jidosha Kabushiki Kaisha, Toyota-shi, Aichi-ken 471-8571 (JP)
(72) Inventor: TAKAI, Tomohisa, Toyota-shi, Aichi-ken 471-8571 (JP); YAMAGUCHI, Yuhei, Toyota-shi, Aichi-ken 471-8571 (JP); TOYOSHIMA, Satoshi, Toyota-shi, Aichi-ken 471-8571 (JP); WATANABE, Yuta, Toyota-shi, Aichi-ken 471-8571 (JP); HONDA, Mikio, Toyota-shi, Aichi-ken 471-8571 (JP); ODA, Shiro, Toyota-shi, Aichi-ken 471-8571 (JP); TAIRA, Tetsuya, Toyota-shi, Aichi-ken 471-8571 (JP); OTSUKI, Nobuhisa, Toyota-shi, Aichi-ken 471-8571 (JP)
(74) Representative: D Young & Co LLP

(57) **Abstract**

A management system is for managing a mobile robot (100) as an autonomous moving apparatus and includes a cleanliness level setting unit (111), an instruction reception unit (112), a storage unit (190), and a control unit (113). The cleanliness level setting unit (111) sets a cleanliness level of the autonomous moving apparatus. The instruction reception unit (112) receives an instruction for a task the autonomous moving apparatus executes. The storage unit (190) stores a database associating information about at least an operator or a destination of the autonomous moving apparatus, a place which the autonomous moving apparatus passes through, and an object to be conveyed by the autonomous moving apparatus, which are included in the task, with the cleanliness level of the autonomous moving apparatus. The control unit (113) controls the setting of the cleanliness level of the autonomous moving apparatus or processing for the instruction.

## Description

### BACKGROUND

The present disclosure relates to a management system, a management method, a program, and an autonomous moving apparatus.

The development of autonomous moving apparatuses that autonomously move in certain buildings and facilities is advancing. By equipping such an autonomous moving apparatus with a parcel carrier, it can be used as an automatic delivery apparatus that automatically delivers parcels. The automatic delivery apparatus can deliver, for example, a parcel loaded at a starting place to a destination by autonomously traveling from the starting point to the destination.

For example, an automatic delivery apparatus disclosed in United States Patent No. 9,026,301 includes an autonomously movable tractor part and a parcel carrier part. Further, a computer provided in them stores electronic maps of floor plans of buildings and routes that the automatic delivery apparatus follows when it moves from one place to the next place. This automatic delivery apparatus conveys various goods using different types of parcel carrier parts according to the purpose.

### SUMMARY

Since the autonomous moving apparatus conveys various goods, the state of the good to be conveyed may or may not be clean. However, for example, it may not be preferable in terms of sanitary control to convey goods with a high sanitary control level after conveying goods with a low sanitary control level. Further, the autonomous moving apparatus moves in various environments which may or may not be clean. However, it may not be preferable in terms of sanitary control for the autonomous moving apparatus to move in an environment with a low sanitary control level and then move in an environment with a high sanitary control level.

The present disclosure has been made to solve such a problem, and provides a management system and the like preferable in terms of sanitary control.

An example aspect of the present disclosure is a management system for managing an autonomous moving apparatus that includes a cleanliness level setting unit, an instruction reception unit, a storage unit, and a control unit. The cleanliness level setting unit is configured to set a cleanliness level of the autonomous moving apparatus. The instruction reception unit is configured to receive an instruction for a task executed by the autonomous moving apparatus. The storage unit is configured to store a database, in which the database associates information about at least one of an operator of the autonomous moving apparatus, a destination of the autonomous moving apparatus, a place which the autonomous moving apparatus passes through, and an object to be conveyed by the autonomous moving apparatus, which are included in the task, with the cleanliness level of the autonomous moving apparatus. The control unit is configured to control the setting of the cleanliness level of the autonomous moving apparatus or processing for the instruction based on the information included in the database.

Thus, the management system, for example, executes the task corresponding to the set cleanliness level or sets the cleanliness level corresponding to the instructed task. That is, the management system can execute the task corresponding to the cleanliness level set in the autonomous moving apparatus.

In the above management system, the control unit may be configured to determine a content of the task received by the autonomous moving apparatus based on the set cleanliness level. Thus, the operability of the management system can be improved while maintaining the set cleanliness level.

In the above management system, when the control unit executes the task related to the instruction, the control unit may be configured to set a route corresponding to the cleanliness level associated with the task. Thus, it is possible to prevent the autonomous moving apparatus from being exposed to an environment that does not correspond to the set cleanliness level.

In the above management system, when the control unit executes the task related to the instruction, the control unit may be configured to set an operation acceleration corresponding to the cleanliness level associated with the task. Thus, for example, the management system can efficiently convey the object to be conveyed in accordance with the cleanliness level of the object to be conveyed.

In the above management system, when the control unit executes the task related to the instruction, the control unit may be configured to update the cleanliness level of the autonomous moving apparatus to the cleanliness level associated with the task. Thus, the management system can set the cleanliness level according to the executed task.

In the above management system, after a first task associated with a first cleanliness level is executed, the control unit may be configured not to allow an execution of a second task associated with a second cleanliness level different from the first cleanliness level. Thus, the management system can prevent unintentional changes of the cleanliness level, thereby preventing the autonomous moving apparatus from being exposed to an environment not corresponding to the cleanliness level set in the autonomous moving apparatus.

Further, the above management system may further include an environmental monitor configured to set a cleanliness level of a surrounding environment of the autonomous moving apparatus. In this case, the control unit may be configured to update, when the autonomous moving apparatus is exposed to the surrounding environment associated with a fourth cleanliness level different from a third cleanliness level as a result of executing a third task associated with the third cleanliness level, the cleanliness level of the autonomous moving apparatus to the fourth cleanliness level. Thus, the management system can dynamically respond to a change in the cleanliness level by executing a predetermined task.

Another example aspect of the present disclosure is an autonomous moving apparatus including the management system according to any one of the above-described management systems, an operation reception unit, and a drive unit. The operation reception unit is configured to receive the instruction for the task. The drive unit is configured to move the autonomous moving apparatus according to the task. Thus, the autonomous moving apparatus can manage its own cleanliness level.

Another example aspect of the present disclosure is a management method for managing an autonomous moving apparatus. The management method includes a cleanliness level setting step, an instruction receiving step, a storing step, and a control step. In the cleanliness level setting step, a cleanliness level of the autonomous moving apparatus is set. In the instruction receiving step, an instruction for a task executed by the autonomous moving apparatus is received. In the storing step, a database is stored. The database associates information about at least one of an operator of the autonomous moving apparatus, a destination of the autonomous moving apparatus, a place which the autonomous moving apparatus passes through, and an object to be conveyed by the autonomous moving apparatus, which are included in the task, with the cleanliness level of the autonomous moving apparatus. In the control step, the cleanliness level of the autonomous moving apparatus or processing for the instruction is stored based on the information stored in the database.

Thus, the management method, for example, executes the task corresponding to the set cleanliness level or sets the cleanliness level corresponding to the instructed task. That is, the management method can cause the autonomous moving apparatus to execute the task corresponding to the cleanliness level set in the autonomous moving apparatus.

Another example aspect of the present disclosure is a management program for executing a management method for managing an autonomous moving apparatus. The management method includes a cleanliness level setting step, an instruction receiving step, a storing step, and a control step. In the cleanliness level setting step, a cleanliness level of the autonomous moving apparatus is set. In the instruction receiving step, an instruction for a task executed by the autonomous moving apparatus is received. In the storing step, a database is stored. The database associates information about at least one of an operator of the autonomous moving apparatus, a destination of the autonomous moving apparatus, a place which the autonomous moving apparatus passes through, and an object to be conveyed by the autonomous moving apparatus, which are included in the task, with the cleanliness level of the autonomous moving apparatus. In the control step, the cleanliness level of the autonomous moving apparatus or processing for the instruction is stored based on the information stored in the database.

Thus, the program, for example, executes the task corresponding to the set cleanliness level or sets the cleanliness level corresponding to the instructed task. That is, the program can cause the autonomous moving apparatus to execute the task corresponding to the cleanliness level set in the autonomous moving apparatus.

According to the present disclosure, it is possible to provide a management system and the like preferable in terms of sanitary control.

The above and other objects, features and advantages of the present disclosure will become more fully understood from the detailed description given hereinbelow and the accompanying drawings which are given by way of illustration only, and thus are not to be considered as limiting the present disclosure.

### BRIEF DESCRIPTION OF DRAWINGS

Fig. 1 is an overview of a mobile robot according to a first embodiment;
Fig. 2 is a block diagram of a mobile robot according to the first embodiment;
Fig. 3 is a table showing an example of a database related to a cleanliness level;
Fig. 4 is a flowchart showing an operation of a mobile robot according to the first embodiment;
Fig. 5 is a first diagram showing an example of an operation screen of the mobile robot according to the first embodiment;
Fig. 6 is a second diagram showing an example of an operation screen of the mobile robot according to the first embodiment;
Fig. 7 is a flowchart showing an operation of a mobile robot according to a second embodiment;
Fig. 8 is a first diagram showing an example of an operation screen of the mobile robot according to the second embodiment;
Fig. 9 is a second diagram showing an example of the operation screen of the mobile robot according to the second embodiment;
Fig. 10 is a block diagram of a management system according to a third embodiment;
Fig. 11 is a table showing an example of a cleanliness level setting in an overall control unit according to the third embodiment; and
Fig. 12 is a flowchart showing an operation of the management system according to the third embodiment.

### DESCRIPTION OF EMBODIMENTS

Hereinafter, the present disclosure will be explained through embodiments of the present disclosure. However, they are not intended to limit the scope of the present disclosure according to the claims. Further, all of the components/structures described in the embodiments are not necessarily indispensable as means for solving the problem. For clarifying the explanation, the following description and the drawings are partially omitted and simplified as appropriate. The same symbols are assigned to the same elements throughout the drawings and duplicated explanations are omitted as appropriate.

### [First Embodiment]

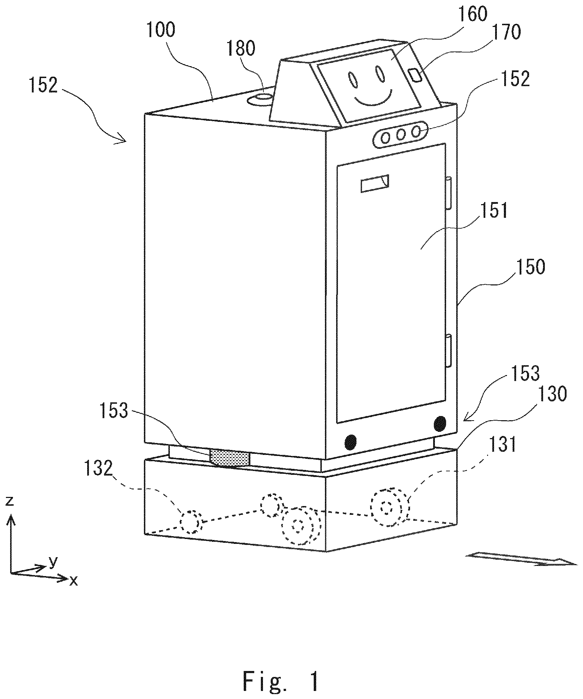

A first embodiment will be described below with reference to the drawings. Fig. 1 is an overview of a mobile robot according to the first embodiment. A mobile robot 100 shown in Fig. 1 is an embodiment of an autonomous moving apparatus that autonomously moves in a predetermined facility. Note that in Fig. 1, a right-handed orthogonal coordinate system is shown for the sake of convenience for explaining a positional relation among components.

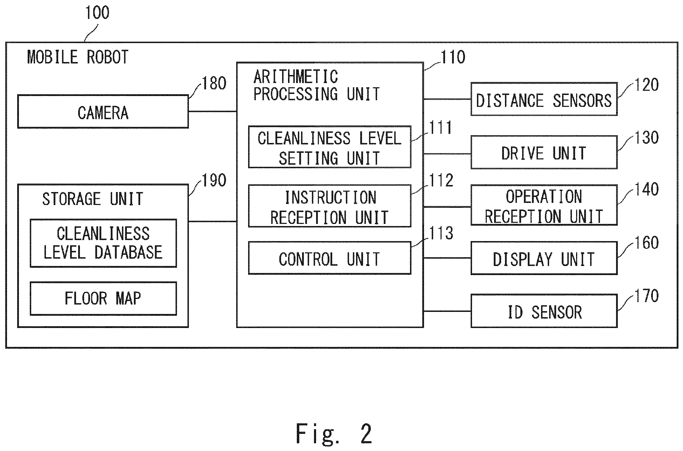

The mobile robot 100 is a moving object whose rectangular-parallelepiped main body moves on the floor surface which serves as the surface on which the mobile robot 100 moves. The mobile robot 100 includes a drive unit 130 as moving means. The drive unit 130 includes two drive wheels 131 that are in contact with the floor surface and configured so as to be able to independently rotate about one rotation axis extending in a direction (a lateral direction or the y-axis direction in Fig. 2) perpendicular to a straight-traveling direction (a longitudinal direction or the x-axis direction in Fig. 2), and casters 132 that are in contact with the floor surface. The mobile robot 100 moves forward or backward by driving the left and right drive wheels 131 at the same rotational speed, and turns (or rotates) by driving the left and right drive wheels 131 at different rotational speeds or in different rotational directions.

The mobile robot 100 includes a housing part 150 above the drive unit 130. The housing part 150 includes a storage cabinet door 151. When the storage cabinet door 151 is opened, a storage cabinet for storing certain objects to be conveyed is provided inside the housing part 150. That is, the mobile robot 100 can be regarded as a conveying robot for conveying a predetermined good.

Front and rear distance sensors 152 are provided above the storage cabinet door 151 and also on a surface opposite to a surface where the storage cabinet door 151 is provided (i.e., the respective surfaces of the mobile robot 100 in a longitudinal direction) of the housing part 150. The front and rear distance sensors 152 can detect a distance between the mobile robot 100 and an object around the mobile robot 100 by detecting the object. The front and rear distance sensor 152 measures a distance to the object included in image data of an image photographed by, for example, a stereo camera and an infrared scanner. Left and right distance sensors 153 are provided at lower parts of the respective surfaces of the housing part 150 in the lateral direction. The left and right distance sensors 153 are distance measuring sensors using a laser beam, a millimeter wave, a near infrared ray, or the like, and can detect a distance to an object around the mobile robot 100. The front and rear distance sensors 152 are provided on the respective surfaces of the housing part 150 in the longitudinal direction. By providing the left and right distance sensors 153 on the respective surfaces of the housing part 150 in the lateral direction, the mobile robot 100 can detect a distance to the object in all directions.

A rectangular protruding part is provided on an upper surface of the housing part 150, and a display unit 160 is disposed on the protruding part. The display unit 160 is a display unit including, for example, a liquid crystal panel, and displays various information of the mobile robot 100. A touch panel for receiving an operation from a user is disposed over the display unit 160.

An ID sensor 170 is provided beside the display unit 160. The ID sensor 170 identifies an ID (Identification) of a user who operates the mobile robot 100, and detects a unique identifier included in an ID card possessed by each user. The ID sensor 170 includes an antenna for reading, for example, information of a radio tag. By bringing the ID card close to the ID sensor 170, the user makes the mobile robot 100 recognize the ID of the user who is an operator.

A camera 180 is also installed on an upper surface of the housing part 150. The camera 180 includes, for example, an objective lens facing an area to be photographed and an image sensor for generating image data of an image to be photographed. The camera 180 is disposed so as to photograph the upper part of the mobile robot 100. Thus, the mobile robot 100 can photograph, for example, the ceiling of a building in which the mobile robot 100 is moving. When a display for specifying a place is shown on the ceiling, the mobile robot can recognize information included in the display by photographing the display of the ceiling.

Next, a system configuration of the mobile robot 100 will be described with reference to Fig. 2. Fig. 2 is a block diagram of the mobile robot according to the first embodiment. The mobile robot 100 includes, as main components, an arithmetic processing unit 110, distance sensors 120, a drive unit 130, an operation reception unit 140, a display unit 160, an ID sensor 170, a camera 180, and a storage unit 190.

The arithmetic processing unit 110 is an information processing apparatus including an arithmetic unit such as a CPU (Central Processing Unit). The arithmetic processing unit 110 includes hardware included in the arithmetic processing unit 110 and a program stored in the hardware. That is, the processing executed by the arithmetic processing unit 110 is achieved by hardware or software. The arithmetic processing unit 110 includes a cleanliness level setting unit 111, an instruction reception unit 112, and a control unit 113.

The cleanliness level setting unit 111 sets a cleanliness level of the mobile robot 100. The cleanliness level is an index associated with contents of a task executed by the mobile robot 100, and is defined by, for example, a predetermined character string. The cleanliness level setting unit 111 includes a non-volatile memory and stores the cleanliness level set in the mobile robot 100. The cleanliness level setting unit 111 stores the cleanliness level to be set according to an instruction of the control unit 113. The cleanliness level setting unit 111 outputs a value of the cleanliness level set in response to a request from the control unit 113. Information included in the non-volatile memory may include information indicating whether the cleanliness level is defined.

The instruction reception unit 112 receives an instruction for the autonomous moving apparatus to execute a predetermined task. The instruction reception unit 112 receives a task designated by the user through the operation reception unit 140 and the display unit 160 as an instruction. The instruction reception unit 112 supplies the instruction for the received task to the control unit 113.

The control unit 113 acquires various pieces of information from each component and instructs each component according to the acquired information. For example, the control unit 113 detects a position of the mobile robot 100 from information of a position acquired from the camera 180, information of an object around the mobile robot 100 acquired from the distance sensors 120, and so on. Then, the control unit 113 calculates a route to a destination from the detected position, and instructs the drive unit 130 to move along the route according to the calculated route. When such processing is executed, the control unit 113 refers to information related to a floor map stored in the storage unit 190.

The control unit 113 controls a setting of the cleanliness level of the mobile robot 100 or processing for the instruction based on information included in a database. More specifically, for example, the control unit 113 instructs the cleanliness level setting unit 111 to set or update the cleanliness level in response to an instruction received from the instruction reception unit 112. The control unit 113 acquires the set cleanliness level from the cleanliness level setting unit 111, and determines whether to execute the task in accordance with the acquired cleanliness level and the instruction received from the instruction reception unit 112. The control unit 113 refers to the database related to the cleanliness level stored in the storage unit 190 when the above processing is performed.

The term the distance sensors 120 collectively indicates the sensors for acquiring distance data about a distance between an object around the mobile robot 100 and the mobile robot 100. The distance sensors 120 include the front and rear distance sensors 152 and the left and right distance sensors 153 shown in Fig. 1. Each of the sensors included in the distance sensors 120 supplies the distance data detected by the sensors to the arithmetic processing unit 110. The drive unit 130 is connected to the arithmetic processing unit 110 and drives the drive wheels 131 in response to an instruction from the arithmetic processing unit 110.

The operation reception unit 140 receives an input operation from a user and transmits an operation signal to the arithmetic processing unit 110. As means for receiving an input operation from a user, the operation reception unit 140 includes, for example, operation buttons, a touch panel disposed over the display unit 160, etc. For example, a user turns on/off a power supply, inputs a task, or opens/closes the storage cabinet door 151 by operating the above-described input operation means.

The display unit 160 is connected to the arithmetic processing unit 110 to display the image data received from the arithmetic processing unit 110. When the operation reception unit 140 receives an operation on the touch panel disposed over the display unit 160, the display unit 160 displays information in association with the operation reception unit 140.

The ID sensor 170 is connected to the arithmetic processing unit 110 and supplies a detected signal to the arithmetic processing unit 110. The camera 180 is connected to the arithmetic processing unit 110 and supplies the photographed image data to the arithmetic processing unit 110.

The storage unit 190 includes a non-volatile memory such as a flash memory or an SSD (Solid State Drive), and stores, for example, a database related to a cleanliness level. This database stored in the storage unit 190 associates information related to at least one of an operator of the autonomous moving apparatus, a destination of the autonomous moving apparatus, a place which the autonomous moving apparatus passes through, or an object to be conveyed by the autonomous moving apparatus, which are included in the task, with the cleanliness level of the autonomous moving apparatus. The storage unit 190 stores the floor map of a facility used by the mobile robot 100 in order to autonomously move. The storage unit 190 is connected to the arithmetic processing unit 110, and supplies the stored information to the arithmetic processing unit 110 in response to a request from the arithmetic processing unit 110.

In the above-described configuration of the mobile robot 100, the configuration included in the arithmetic processing unit 110 and the storage unit 190 is referred to as a management system of the mobile robot 100. The management system of the mobile robot 100 includes at least the cleanliness level setting unit 111, the instruction reception unit 112, the control unit 113, and the storage unit 190. With such a configuration, for example, the management system executes the task corresponding to the set cleanliness level or sets the cleanliness level corresponding to the instructed task. That is, the management system can execute a task corresponding to the cleanliness level set in the mobile robot 100.

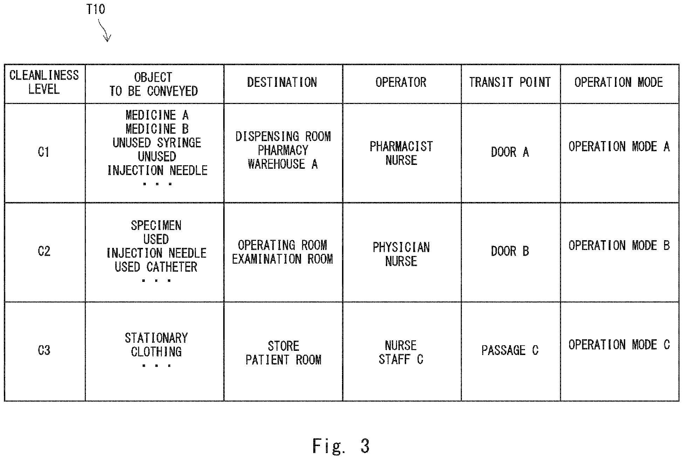

Next, a database related to the cleanliness level stored in the storage unit 190 will be described with reference to Fig. 3. Fig. 3 is a table showing an example of the database related to the cleanliness levels. The table shown in Fig. 3 has four vertical rows and six horizontal columns. For convenience of explanation, the upper left cell of a table T10 is defined as a first column of a first row, and the lower right cell is defined as a sixth column of a fourth row. The table T10 shows, from the first column to the sixth column of the first row, items of the cleanliness level, an object to be conveyed, destination, operator, transit point, and operation mode. The table T10 shows that these items on the same row are associated with each other.

For example, C1 is written in the first column of the second row of the table T10. This indicates that the cleanliness level corresponding to the second row is C1. In the second column of the second row, medicine A, medicine B, unused syringe, unused injection needle, etc. are written. This indicates that the object to be conveyed corresponding to the cleanliness level C1 is written in the second column of the second row. Likewise, in the third column of the second row, dispensing room, pharmacy, and warehouse A are written as destinations corresponding to the cleanliness level C1. That is, the object to be conveyed corresponding to the cleanliness level C1 is, for example, a medicine and an unused instrument, and the destinations corresponding to the cleanliness level C1 is a dispensing room and a pharmacy. Further, the table T10 shows that the operators corresponding to the cleanliness level C1 are a pharmacist and a nurse, the transit point corresponding to the cleanliness level C1 is a door A, and an operation mode corresponding to the cleanliness level C1 is an operation mode A.

Likewise, the table T10 shows that the items corresponding to the cleanliness level C2 are the object to be conveyed (specimen, used injection needle, used catheter, etc.), the destination (operating room and examination room), the operator (physician and nurse), the transit point (door B), and the operation mode (operation mode B) shown in the third row. The table T10 shows that the items corresponding to the cleanliness level C3 are the object to be conveyed (stationery and clothing), the destination (store and patient room), the operator (nurse and staff C), the transit point (passage C), and the operation mode (operation mode C) shown in the fourth row.

In the table T10, the operation mode is an operation parameter related to acceleration, deceleration, and turning performed by the mobile robot 100. For example, the operation acceleration when the mobile robot 100 moves in the operation mode A is set smaller than the operation acceleration when the mobile robot 100 moves in the operation mode C. With such a setting, the mobile robot 100 can perform efficient conveyance corresponding to the cleanliness level of the object to be conveyed in accordance with the cleanliness level of the object to be conveyed.

The mobile robot 100 refers to the database shown in the table T10 to set the cleanliness level and process the instructed task. For example, when the cleanliness level C1 is set in the mobile robot 100, the mobile robot 100 can receive an operation of a pharmacist and convey the medicine A to the warehouse A. In this case, the mobile robot 100 may pass through the door A as a transit point. The mobile robot 100 performs a conveyance operation in the operation mode A. Furthermore, for example, when the cleanliness level C1 is set in the mobile robot 100, the mobile robot 100 does not receive an operation of a staff C. Alternatively, for example, when the cleanliness level C1 is set in the mobile robot 100, the mobile robot 100 may update the cleanliness level to C3 instead of receiving an operation from the staff C. By such management in accordance with the defined cleanliness level, the mobile robot 100 can perform tasks while preventing contamination and pollution that may cause problems in sanitation or safety.

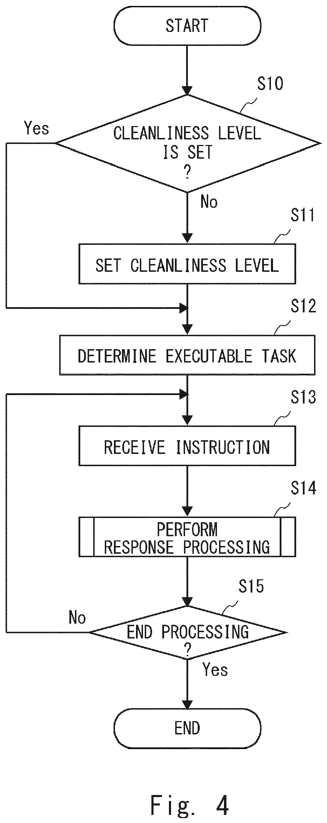

Next, processing performed by the mobile robot 100 will be described with reference to Fig. 4. Fig. 4 is a flowchart showing the operation of the mobile robot 100 according to the first embodiment. The flowchart shown in Fig. 4 shows processing performed by the arithmetic processing unit 110 of the mobile robot 100. The flowchart shown in Fig. 4 starts, for example, when the mobile robot 100 is activated as trigger.

First, the arithmetic processing unit 110 determines whether the cleanliness level is set (Step S10). More specifically, for example, the control unit 113 inquires the cleanliness level setting unit 111 whether the cleanliness level is set. In response to the inquiry from the control unit 113, when the cleanliness level is not set, the cleanliness level setting unit 111 sends a reply that the cleanliness level is not set, whereas when the cleanliness level is set, the cleanliness level setting unit 111 sends the set cleanliness level. That is, when the arithmetic processing unit 110 determines that the cleanliness level is set (Step S10: Yes), the process proceeds to Step S12.

On the other hand, when it is not determined that the cleanliness level is set (Step S10: No), the arithmetic processing unit 110 sets the cleanliness level (Step S11). In the setting of the cleanliness level when the cleanliness level is not set, the arithmetic processing unit 110 may be configured to receive a designation of the cleanliness level from the user. In this case, the arithmetic processing unit 110 may set one predetermined cleanliness level from among C1, C2, and C3.

Next, the arithmetic processing unit 110 determines an executable task (Step S12). More specifically, the arithmetic processing unit 110 refers to the database related to the cleanliness level stored in the storage unit 190 and reads items corresponding to the cleanliness level set in the mobile robot 100.

Next, the arithmetic processing unit 110 receives an instruction from the user (Step S13). The instruction from the user includes a content to instruct an execution of a predetermined task. The arithmetic processing unit 110 receives this instruction through the operation reception unit 140.

Next, the arithmetic processing unit 110 performs response processing in response to the received instruction (Step S14). The response processing is, for example, an execution of a task related to the received instruction. When the task is executed, the arithmetic processing unit 110 refers to the information and the floor map included in the database related to the cleanliness level stored in the storage unit 190.

When the response processing is ended, such as when the execution of the task related to the received instruction is completed, the arithmetic processing unit 110 determines whether to end a series of processing (Step S15). When an operation such as shutting down the system is received, the arithmetic processing unit 110 determines that the series of processing is to be ended (Step S15: Yes), and ends the processing. On the other hand, when it is not determined that the series of processing is to be ended (Step S15: No), the arithmetic processing unit 110 returns to Step S13 to receive the next instruction.

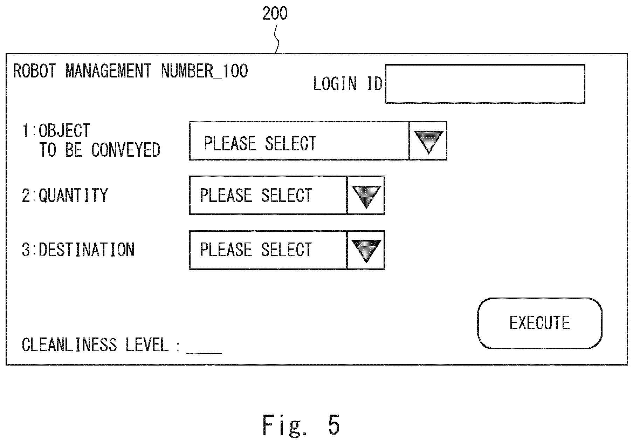

Next, a display example of the display unit 160 will be described with reference to Fig. 5. Fig. 5 is a first diagram showing an example of an operation screen of the mobile robot 100 according to the first embodiment. An image 200 shown in Fig. 5 is a screen for receiving an instruction for a task to be executed by the mobile robot 100 from among the operation screens displayed on the display unit 160.

On the upper left of the image 200, "Robot management number _ 100" is displayed as a management number of the mobile robot. A field for displaying a login ID of the operator is disposed in the upper right of the image 200. In the middle of the image 200, "1: Object to be conveyed", "2: Quantity", and "3: Destination" are displayed, and a selectable list is disposed on the right side of each field together with the message "Please select". The user can display the selectable items by touching the selectable list. On the lower left of the image 200, "Cleanliness level: _" is displayed. When the cleanliness level is set in the mobile robot 100, the set cleanliness level is displayed in the underlined part on the right side of "Cleanliness level:".

A button labeled "Execute" is displayed as an icon in the lower right of the image 200. The mobile robot 100 receives an input of an instruction related to the task, and when it is determined that the received task is executable, the mobile robot 100 starts executing the received task upon a detection of a touch of the execution button by the user.

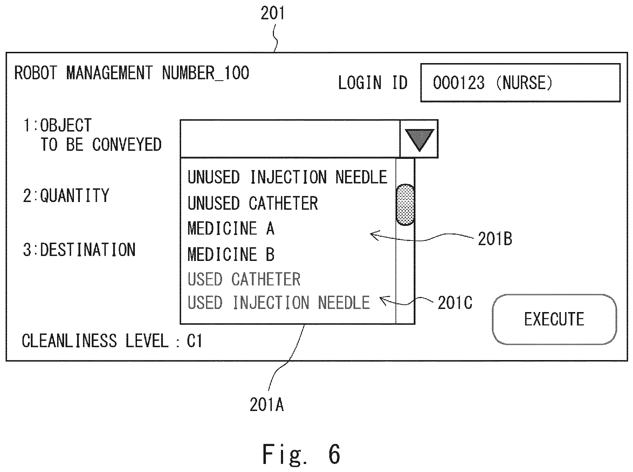

Next, an example of an instruction received by the mobile robot 100 will be described with reference to Fig. 6. Fig. 6 is a second diagram showing an example of an operation screen of the mobile robot according to the first embodiment. An image 201 shown in Fig. 6 shows a situation in which the mobile robot 100 receives an instruction for a task from the user. The cleanliness level of the mobile robot 100 in the image 201 is set to C1. The mobile robot 100 is receiving an operation from a user who is a nurse and has the login ID 000123, and is receiving an input related to an object to be conveyed from the user. In the example shown in the drawing, the execution button shown in the lower right of the image 201 is not in an effective state. Therefore, the execute button is grayed out.

A selection screen 201A shown in the drawing is a screen displayed when a touch of the selection field of the object to be conveyed is detected. In the selection screen 201A, the items of the objects to be conveyed shown in the table T10 in Fig. 3 are listed. The selection screen 201A is configured in such a way that the display thereof scrolls up and down when swiped by the user.

The selection screen 201A includes a selectable part 201B and an unselectable part 201C. The selectable part 201B is a part for displaying an instruction that can be received by the mobile robot 100, and the characters of the items to be listed are displayed in a dark color. The unselectable part 201C is a part for displaying an instruction that the mobile robot 100 does not receive, and the characters of the items to be listed are grayed out and displayed in a light color.

For example, in the selection screen 201A shown in the drawing, "unused injection needle", "unused catheter", "medicine A", and "medicine B" are included in the selectable part 201B. These items correspond to the cleanliness level C1 set for the mobile robot 100 in the image 201 and are included in the second column of the second row shown in the table T10. In the selection screen 201A shown in the drawing, "used catheter" and "used injection needle" are included in the unselectable part 201C. These items do not correspond to the cleanliness level C1. For this reason, in the example shown in the drawing, these items cannot be selected by the user.

In this way, the control unit 113 of the mobile robot 100 determines the contents of the task received by the mobile robot 100 in accordance with the cleanliness level to be set. Then, the control unit 113 sets an instruction related to a task to be received so that it becomes selectable by the user, and sets an instruction other than the instruction related to the task to be received so that it becomes unselectable. Thus, the operability of the mobile robot 100 can be improved while maintaining the set cleanliness level.

In the same manner as the above example, in regard to the items related to the destination, the control unit 113 sets the item related to the set cleanliness level so that it becomes selectable, and sets an item other than the item related to the set cleanliness level so that it becomes unselectable. In the examples shown in Figs. 5 and 6, although information about the transit point and information about the operation mode are not displayed, the control unit 113 sets the transit point and the operation mode in accordance with the set cleanliness level.

For example, when the cleanliness level set in the mobile robot 100 is C1, the control unit 113 sets the door A as the transit point. In this manner, by setting the transit point in accordance with the cleanliness level, it is possible to prevent the mobile robot 100 from being exposed to an environment that does not correspond to the set cleanliness level.

For example, when the cleanliness level set in the mobile robot 100 is C1, the control unit 113 sets the operation mode A as the operation mode. By doing so, for example, the mobile robot 100 can efficiently convey the object to be conveyed in accordance with the cleanliness level of the object to be conveyed.

Although the first embodiment has been described above, the mobile robot 100 according to the first embodiment is not limited to the above-described configuration. For example, the cleanliness level is not limited to the three levels described above. The cleanliness level may be categorized into two or four or more levels. Further, some of the items shown in the second to sixth columns from among the items shown in Fig. 3 may be omitted. For example, the mobile robot 100 may be a mobile robot that does not convey an object to be conveyed. The instruction for the task corresponding to the cleanliness level described above is an example, and the correspondence relationship between the cleanliness level and the instruction for the task is not limited to the above contents. The definition of cleanliness level may comply with international and national standards for sanitary control.

The mobile robot 100 described above includes a management system. The management system, for example, executes the task corresponding to the set cleanliness level or sets the cleanliness level corresponding to the instructed task. That is, the management system can execute a task corresponding to the cleanliness level set in the mobile robot 100. By equipping the mobile robot 100 with the above management system, the mobile robot 100 can manage its own cleanliness level. Therefore, according to the first embodiment, it is possible to provide a management system and the like favorable in terms of sanitary control.

### [Second embodiment]

Next, a second embodiment will be described. A mobile robot 100 according to the second embodiment differs from that according to the first embodiment in the processing for receiving an instruction related to a task and the processing for setting a cleanliness level.

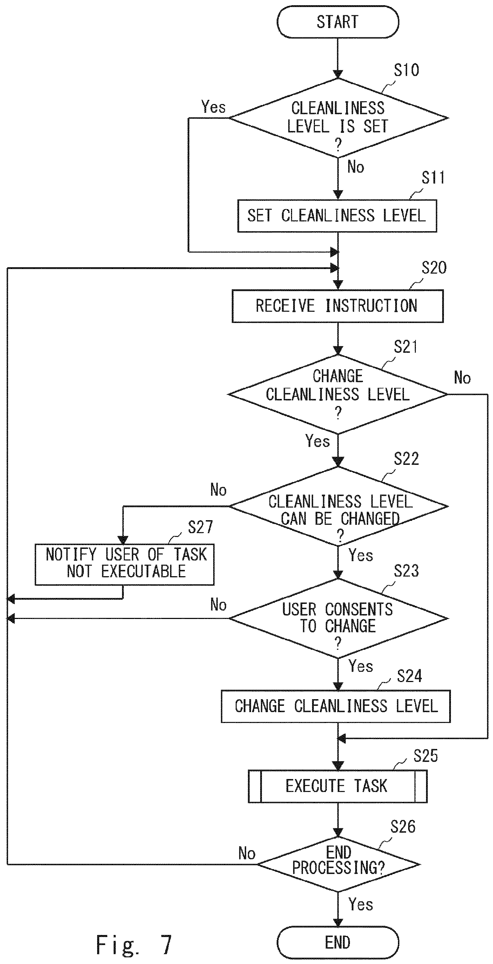

The processing of the mobile robot according to the second embodiment will be described with reference to Fig. 7. Fig. 7 is a flowchart showing an operation of the mobile robot according to the second embodiment. The flowchart shown in Fig. 7 is processing performed by the arithmetic processing unit 110, and Steps S10 and S11 are the same as those in the first embodiment.

After Step S11, the arithmetic processing unit 110 receives an instruction related to the task (Step S20). Here, the arithmetic processing unit 110 receives not only the instruction corresponding to the set cleanliness level but also the instruction not corresponding to the set cleanliness level. That is, for example, when the cleanliness level C1 is set in the mobile robot 100, the arithmetic processing unit 110 receives not only an instruction corresponding to the cleanliness level C1 but also an instruction corresponding to the cleanliness levels C2 and C3.

Next, the arithmetic processing unit 110 determines whether the cleanliness level is changed when the received instruction is executed (Step S21). When it is determined that the cleanliness level is changed when the received instruction is executed (Step S21: Yes), the arithmetic processing unit 110 proceeds to Step S22. On the other hand, when it is determined that the cleanliness level is not changed when the received instruction is executed (Step S21: No), the process proceeds to Step S25.

In Step S22, the arithmetic processing unit 110 determines whether the cleanliness level can be changed (Step S22). The case where the cleanliness level can be changed is, for example, a case where the cleanliness level is changed from C1 to C2, or a case where the cleanliness level is changed from C1 to C3. For example, when the mobile robot 100 which has conveyed the medicine and instruments corresponding to the cleanliness level C1 is made to convey the stationery and the like corresponding to the cleanliness level C3, no sanitary control problem occurs. Therefore, the arithmetic processing unit 110 is configured to be able to receive an instruction corresponding to the cleanliness level C3 when the cleanliness level C1 is set.

On the other hand, the case where the cleanliness level cannot be changed is, for example, a case where the cleanliness level is changed from C2 to C1, or a case where the cleanliness level is changed from C3 to C1. For example, when the mobile robot 100 is made to convey a medicine or the like corresponding to the cleanliness level C1 after conveying a specimen corresponding to the cleanliness level C2, the medicine to be conveyed may be contaminated with the residue of the specimen. In addition, when the mobile robot 100 is made to convey a medicine or the like corresponding to the cleanliness level C1 after conveying stationery or the like corresponding to the cleanliness level C3, a suspended substance in the air such as dust may attach to the mobile robot 100 to be brought into the dispensing room, because an environment in which stationery or the like is conveyed has a lower sanitary control level than that of an environment where medicines are prepared. Therefore, the arithmetic processing unit 110 is set such that the cleanliness level cannot be changed in such a case.

When it is determined in Step S22 that the cleanliness level can be changed (Step S22: Yes), the arithmetic processing unit 110 proceeds to Step S23. On the other hand, when it is not determined that the cleanliness level can be changed (Step S22: No), the arithmetic processing unit 110 notifies the user that the execution of the task related to the received instruction cannot be allowed (Step S27), and the process returns to Step S20.

In Step S23, the arithmetic processing unit 110 inquires the user whether the cleanliness level may be changed (Step S23). When it is determined that the user consents to the change in the cleanliness level (Step S23: Yes), the arithmetic processing unit 110 proceeds to Step S24 to change the cleanliness level (Step S24). On the other hand, when it is not determined that the user consents to the change in the cleanliness level (Step S23: No), the arithmetic processing unit 110 returns to Step S20.

In Step S25, the arithmetic processing unit 110 executes the task corresponding to the received instruction (Step S25). When the execution of the task is completed, the arithmetic processing unit 110 determines whether to end the series of processing (Step S26). When an operation such as shutting down the system is received, the arithmetic processing unit 110 determines that the series of processing is to be ended (Step S26: Yes), and ends the processing. On the other hand, when it is not determined that the series of processing is to be ended (Step S26: No), the arithmetic processing unit 110 returns to Step S20 to receive the next instruction.

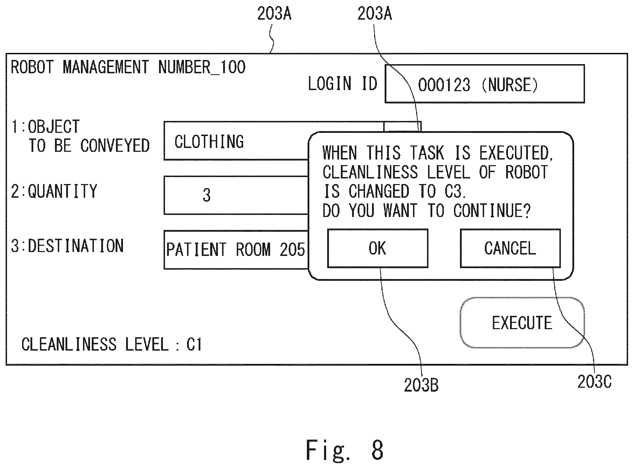

Next, a first example of an operation screen in the processing according to the second embodiment will be described with reference to Fig. 8. Fig. 8 is a first diagram showing an example of an operation screen of the mobile robot according to the second embodiment. An image 203 shown in Fig. 8 shows an example in which the user's consent is obtained in Step S23. A message box 203A for inquiring the user's consent is disposed over the image 203 on the operation screen.

As shown in the drawing, the cleanliness level of the mobile robot 100 in the image 203 is set to C1. Further, the user selects to convey the object to be conveyed "Clothing" to the destination "Patient room 205" for the mobile robot 100. When the task of conveying the object to be conveyed "Clothing" to the destination "Patient room 205", the mobile robot 100 refers to the database related to the cleanliness level of the table T10 and determines that the object to be conveyed passes through the passage C as a transit point. Thus, the cleanliness level of the mobile robot 100 is changed to C3. Then, on the message box 203A included in the image 203, an OK button 203B and a cancel button 203C, as selection buttons for inquiring whether to consent, are displayed together with the message "When this task is executed, cleanliness level of robot is changed to C3. Do you want to continue?". When the selection of the OK button 203B is detected, the arithmetic processing unit 110 proceeds from Step S23 to Step S24. On the other hand, when the selection of the cancel button 203C is detected, the arithmetic processing unit 110 proceeds to Step S20.

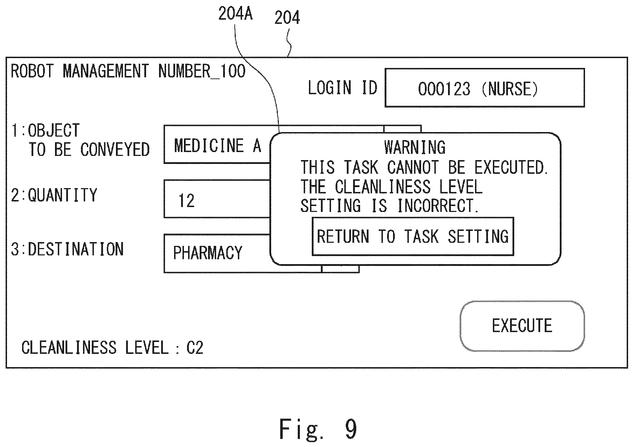

Next, a second example of an operation screen in the processing according to the second embodiment will be described with reference to Fig. 9. Fig. 9 is a second diagram showing an example of an operation screen of the mobile robot according to the second embodiment. An image 204 shown in Fig. 9 shows an example in which the user is notified that the task related to the received instruction in Step S27 cannot be executed. A message box 204A is disposed over the image 204 on the operation screen.

As shown in the drawing, the cleanliness level of the mobile robot 100 on the image 204 is set to C2. Further, the user selects to convey the object to be conveyed "Medicine A" to the destination "Pharmacy" for the mobile robot 100. When the task of conveying the object to be conveyed "Medicine A" to the destination "Pharmacy" is executed, the cleanliness level of the mobile robot 100 needs to be C1. For the above reason, the mobile robot 100 set to the cleanliness level C2 cannot be changed to the cleanliness level C1. Therefore, on the message box 204A included in the image 204, a warning "This task cannot be executed. The cleanliness level setting is incorrect" and a button for returning to the task settings are displayed. When a touch of this button by the user is detected, the arithmetic processing unit 110 proceeds to Step S20.

The second embodiment has been described above. The description of the change of the cleanliness level described above shows an example of the cleanliness level according to the second embodiment, and the user can set whether the cleanliness level is changeable. These settings may be set in advance or may be changed after the operation of the mobile robot 100 is started.

As described above, according to the second embodiment, the management system included in the mobile robot 100 can set the cleanliness level according to the executed task by updating the cleanliness level. In addition, when the management system included in the mobile robot 100 makes a setting to disable a change in the cleanliness level to a cleanliness level not corresponding to the cleanliness level not corresponding to the mobile robot 100, the management system can prevent unintentional changes of the cleanliness level, thereby preventing the mobile robot 100 from being exposed to an environment not corresponding to the cleanliness level set in the mobile robot 100.

### [Third Embodiment]

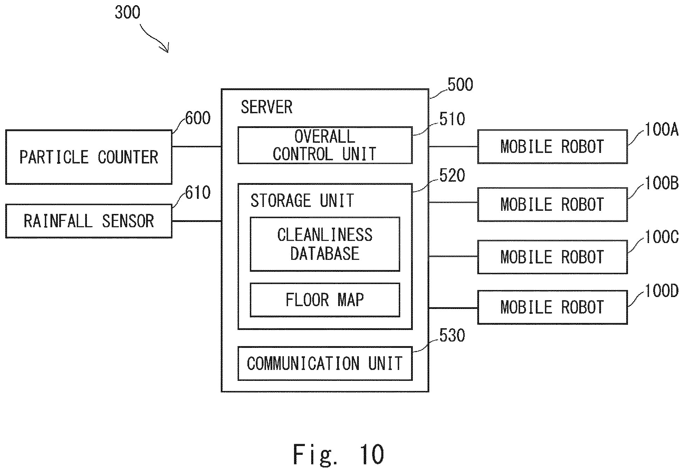

Next, a third embodiment will be described. In the third embodiment, a server manages a plurality of mobile robots. Fig. 10 is a block diagram of a management system according to the third embodiment. A management system 300 shown in the drawing is a system for managing a plurality of mobile robots autonomously moving in a predetermined facility. The management system 300 shown in the drawing includes, as main components, mobile robots 100A to 100 D, a server 500, a particle counter 600, and a rainfall sensor 610.

The mobile robots 100A to 100D are autonomous moving apparatuses that move autonomously within a predetermined site. Each of the mobile robots 100A to 100D individually receives a task and autonomously moves to execute the received task. Each of the mobile robots 100A to 100D has a configuration equivalent to that of the mobile robot 100 shown in, for example, the first or second embodiment and further includes a communication unit. The mobile robots 100A to 100D exchange signals with the server 500 through the communication unit. For example, the mobile robots 100A to 100D notify the server 500 of information about the tasks they are receiving, their own operating statuses, their current positions, or the like. The mobile robots 100A to 100D may receive an instruction from the server 500 as to whether to execute the received task. The communication units included in the mobile robots 100A to 100D may be wireless communication units or wired communication units, but wireless communication units are preferable.

The cleanliness level is set in each of the mobile robots 100A to 100D. Each of the mobile robots 100A to 100D notifies the server 500 of the set cleanliness level. The mobile robots 100A to 100D receive an instruction for changing the cleanliness level from the server 500. When the mobile robots 100A to 100D receive an instruction for changing the cleanliness level from the server 500, they change their cleanliness levels in accordance with the instruction from the server 500.

The server 500 is an apparatus for managing the mobile robots 100A to 100D. The server 500 includes, as main components, an overall control unit 510, a storage unit 520, and a communication unit 530. The server 500 is connected to the mobile robots 100A to 100D so that it can communicate with the mobile robots 100A to 100D. The server 500 is also connected to the particle counter 600 and the rainfall sensor 610.

The overall control unit 510 includes an arithmetic unit such as a CPU (Central Processing Unit), and controls the mobile robots 100A to 100D by executing a predetermined program. The overall control unit 510 includes at least some of the functions of the cleanliness level setting unit 111, the instruction reception unit 112, and the control unit 113 described in the first or second embodiment. Thus, the overall control unit 510 sets the cleanliness level or performs processing corresponding to a task related to the set cleanliness level in cooperation with the mobile robots 100A to 100D.

The overall control unit 510 receives signals related to environmental data of a monitoring place from the particle counter 600 and the rainfall sensor 610, and sets the cleanliness level of the monitoring place based on the received signals. Further, when the mobile robots 100A to 100D have passed through the monitoring place, the overall control unit 510 changes the cleanliness levels of the mobile robots 100A to 100D in accordance with the cleanliness level set at the monitoring place which they have passed through.

The storage unit 520 includes a non-volatile memory such as a flash memory, an SSD (Solid State Drive), or the like, and stores a database related to the cleanliness level which is, for example, equivalent to that described with reference to Fig. 3. The storage unit 520 stores a floor map of a facility used by the mobile robots 100A to 100D in order to autonomously move. The storage unit 520 supplies the stored information to the overall control unit 510 in response to a request from the overall control unit 510.

The communication unit 530 communicates with the mobile robot 100 in a wireless or wired manner. The communication unit 530 supplies information received from the mobile robots 100 to the user, or receives an operation from the user and sends a predetermined instruction to the mobile robots 100A to 100D according to the contents of the received operation. The communication unit 530 may include a router apparatus for performing communication between the server 500 and a plurality of components.

The particle counter 600 is an embodiment of an environment monitor for acquiring environmental data around the mobile robots. The particle counter 600 measures dust as the environmental data in the monitoring place which it is installed, and outputs the measured value to the server 500.

The rainfall sensor 610 is an embodiment of an environmental monitor for acquiring environmental data around the mobile robot. The rainfall sensor 610 detects, for example, rainfall of 1 millimeter or more as the environmental data at the monitoring place which it is installed, and outputs the detected signal to the server 500.

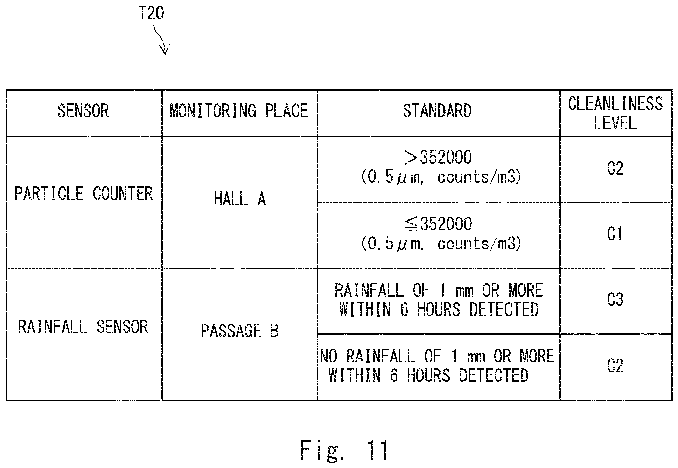

Next, the relationship between the particle counter 600 and the rainfall sensor 610 and the cleanliness level setting will be described with reference to Fig. 11. Fig. 11 is a table showing an example of the cleanliness level setting in the overall control unit according to the third embodiment.

The upper row of a table T20 shown in Fig. 11 shows the monitoring place of the particle counter 600 (hall A), the standard of the environmental data measured by the particle counter 600 (the number of particles of 0.5 µm is more than 352000 counts/m³ and the number of particles of 0.5 µm is 352000 counts / m³ or less), and the cleanliness level (C2 and C1) complying with the standard. The particle counter 600 is installed in the hall A which is the monitoring place, and detects whether the number of particles of 0.5 µm is 35200 or less per cubic meter as the environmental data in the hall A. When the number of particles of 0.5 µm is more than 35200 per cubic meter, the cleanliness level is set to C2. On the other hand, when the number of particles of 0.5 µm is 35200 or less per cubic meter, the cleanliness level is set to C1. For example, the cleanliness level C1 is applied to, for example, a sterile dispensing room where medicines are prepared. The sterile dispensing room requires that the number of particles of 0.5 µm be less than 35200 per cubic meter as the cleaning level of the space.

The hall A is a place which the mobile robots 100A to 100D may pass through. Therefore, in accordance with the environmental data when the mobile robots 100A to 100D pass through the hall A, the overall control unit 510 determines whether the passing mobile robot has been exposed to the environment of the cleanliness level C2 or the environment of the cleanliness level C1.

The lower row of the table T20 shows the monitoring place (passage B) of the rainfall sensor 610, the standard (whether there has been rainfall of 1 mm or more within 6 hours) of the environmental data detected by the rainfall sensor 610, and the cleanliness level (C3 and C2) corresponding to the standard. The rainfall sensor 610 is installed in the passage B, which is a monitoring place, and detects whether there has been rainfall of 1 mm or more within 6 hours as the environmental data in the passage B. When there has been rainfall of 1 mm or more within 6 hours, the cleanliness level of the passage B is set to C3. On the other hand, when there has been no rainfall of 1 mm or more within 6 hours, the cleanliness level of the passage B is set to C2.

The passage B is a place which the mobile robots 100A to 100D may pass through. Therefore, in accordance with the environmental data when the mobile robots 100A to 100D pass through the passage B, the general control unit 510 determines whether the passing mobile robot has been exposed to the environment of the cleanliness level C3 or the environment of the cleanliness level C2.

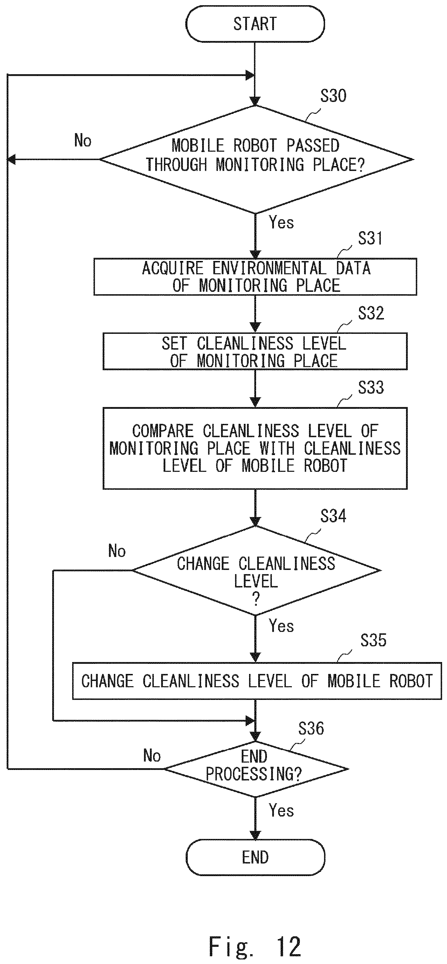

Next, the processing of the management system 300 will be described with reference to Fig. 12. Fig. 12 is a flowchart showing the operation of the management system according to the third embodiment. The flowchart shown in Fig. 12 shows the processing of the overall control unit 510.

First, the overall control unit 510 determines whether any of the mobile robots 100A to 100D passes through the hall A or the passage B, which are the monitoring places (Step S30). For example, the overall control unit 510 designates whether or not the mobile robot passes through the monitoring place by acquiring the position information from each mobile robot. When it is not determined that the mobile robot has passed through the monitoring place (Step S30: No), the overall control unit 510 repeats Step S30. When it is determined that the mobile robot has passed through the monitoring place (Step S30: Yes), the overall control unit 510 acquires the environmental data of the monitoring place through which the mobile robot has passed (Step S31).

Next, the overall control unit 510 sets the cleanliness level of the monitoring place from the acquired environmental data in accordance with the correspondence relationship of the table T20 shown in Fig. 11 (Step S32). Next, the overall control unit 510 compares the set cleanliness level of the monitoring place with the cleanliness level of the mobile robot (Step S33). In this processing, the overall control unit 510 compares the cleanliness level of the monitoring place with the cleanliness level of the mobile robot to determine whether they are the same.

Next, as a result of comparing the set cleanliness level of the monitoring place with the cleanliness level of the mobile robot, the overall control unit 510 determines whether it is necessary to change the cleanliness level of the mobile robot (Step S34). In such processing, when the cleanliness level of the monitoring place and the cleanliness level of the mobile robot are set to be the same, the overall control unit 510 does not determine that it is necessary to change the cleanliness level. When the cleanliness level of the monitoring place and the cleanliness level of the mobile robot are not set to be the same, the overall control unit 510 determines whether or not to change the cleanliness level of the mobile robot in accordance with the cleanliness level of the monitoring place. In this case, the overall control unit 510 determines the cleanliness level, for example, in association with the sanitary control level.

For example, when the mobile robot 100A having the cleanliness level C1 passes through the hall A, the cleanliness level of the hall A is set to C2. In this case, since the mobile robot 100A having the cleanliness level C1 is exposed to the environment C2, the overall control unit 510 determines that the cleanliness level of the mobile robot 100A is changed from C1 to C2. For example, when the mobile robot 100B having the cleanliness level set to C2 passes through the hall A, the cleanliness level of the hall A is set to C1. In this case, since the mobile robot 100B having the cleanliness level C2 is exposed to the environment C1, the overall control unit 510 does not determine that the cleanliness level of the mobile robot 100B is changed from C2 to C1.

When it is determined that the cleanliness level of the mobile robot is changed by the processing shown in the above example (Step S34: Yes), the overall control unit 510 changes the cleanliness level of the mobile robot (Step S35), and then the process proceeds to Step S36. On the other hand, when it is not determined that the cleanliness level of the mobile robot is changed (Step S34: No), the overall control unit 510 proceeds to Step S36.

In Step S36, the overall control unit 510 determines whether or not to end the series of processing (Step S36). When an operation such as shutting down the system is received, the overall control unit 510 determines that the series of processing is to be ended (Step S36: Yes), and ends the processing. On the other hand, when it is not determined that the series of processing is to be ended (Step S36: No), the overall control unit 510 returns to Step S30.

Although the third embodiment has been described above, the management system 300 according to the third embodiment is not limited to the above-described configuration. The configuration shown in Fig. 10 is an example, and the number of mobile robots connected to the server 500 is not limited as long as one or more mobile robots are connected to the server 500. Further, the particle counter 600 and the rainfall sensor 610 are examples for acquiring the environmental data, and the sensors (environmental monitor) for acquiring the environmental data are not limited to them. For example, an image sensor, a hygrometer, or an air pollution measuring instrument may be used as an apparatus for acquiring the environmental data.

The sensor for acquiring the environmental data may be disposed at a predetermined monitoring place or may be disposed in the mobile robot. By disposing a sensor for acquiring the environmental data in the mobile robot, the management system can acquire the environmental data around the mobile robot along the place which the mobile robot moves and perform dynamic processing for the set cleanliness level.

When the cleanliness level of the monitoring place is set, the management system 300 may instruct the mobile robot not to expose the mobile robot not corresponding to the set cleanliness level to the monitoring place. In this case, the management system 300 may dynamically change the contents of the task (e.g., a transit point where the mobile robot passes through) of the mobile robot which do not correspond to the cleanliness level set in the monitoring place so as to correspond to the cleanliness level of the mobile robot.

As described above, according to the third embodiment, it is possible to dynamically respond to a change in the cleanliness level by executing a predetermined task. Therefore, according to the third embodiment, it is possible to provide a management system and the like favorable for sanitary control.

The above program can be stored and provided to a computer using any type of non-transitory computer readable media. Non-transitory computer readable media include any type of tangible storage media. Examples of non-transitory computer readable media include magnetic storage media (such as floppy disks, magnetic tapes, hard disk drives, etc.), optical magnetic storage media (e.g. magneto-optical disks), CD-ROM (Read Only Memory), CD-R (compact disc recordable), CD-R/W (compact disc rewritable), and semiconductor memories (such as mask ROM, PROM (Programmable ROM), EPROM (Erasable PROM), flash ROM, RAM (Random Access Memory), etc.). The program may be provided to a computer using any type of transitory computer readable media. Examples of transitory computer readable media include electric signals, optical signals, and electromagnetic waves. Transitory computer readable media can provide the program to a computer via a wired communication line (e.g. electric wires, and optical fibers) or a wireless communication line.

Note that the present disclosure is not limited to the above-described embodiments, and can be appropriately modified without departing from the scope thereof.

From the disclosure thus described, it will be obvious that the embodiments of the disclosure may be varied in many ways. Such variations are not to be regarded as a departure from the spirit and scope of the disclosure, and all such modifications as would be obvious to one skilled in the art are intended for inclusion within the scope of the following claims.

## Claims

1. A management system for managing an autonomous moving apparatus (100) comprising:
a cleanliness level setting unit (111) configured to set a cleanliness level of the autonomous moving apparatus;
an instruction reception unit (112) configured to receive an instruction for a task executed by the autonomous moving apparatus;
a storage unit (190) configured to store a database, the database associating information about at least one of an operator of the autonomous moving apparatus, a destination of the autonomous moving apparatus, a place which the autonomous moving apparatus passes through, and an object to be conveyed by the autonomous moving apparatus, which are included in the task, with the cleanliness level of the autonomous moving apparatus; and
a control unit (113) configured to control the setting of the cleanliness level of the autonomous moving apparatus or processing for the instruction based on the information included in the database.

2. The management system according to Claim 1, wherein
the control unit is configured to determine a content of the task received by the autonomous moving apparatus based on the set cleanliness level.

3. The management system according to Claim 1 or 2, wherein
when the control unit executes the task related to the instruction, the control unit is configured to set a route corresponding to the cleanliness level associated with the task.

4. The management system according to any one of Claims 1 to 3, wherein
when the control unit executes the task related to the instruction, the control unit is configured to set an operation acceleration corresponding to the cleanliness level associated with the task.

5. The management system according to any one of Claims 1 to 3, wherein
when the control unit executes the task related to the instruction, the control unit is configured to update the cleanliness level of the autonomous moving apparatus to the cleanliness level associated with the task.

6. The management system according to any one of Claims 1 to 5, wherein
after a first task associated with a first cleanliness level is executed, the control unit is configured not to allow an execution of a second task associated with a second cleanliness level different from the first cleanliness level.

7. The management system according to any one of Claims 1 to 6, further comprising an environmental monitor (600,610) configured to set a cleanliness level of a surrounding environment of the autonomous moving apparatus, wherein
the control unit is configured to update, when the autonomous moving apparatus is exposed to the surrounding environment associated with a fourth cleanliness level different from a third cleanliness level as a result of executing a third task associated with the third cleanliness level, the cleanliness level of the autonomous moving apparatus to the fourth cleanliness level.

8. An autonomous moving apparatus comprising:
the management system according to any one of Claims 1 to 7;
an operation reception unit configured to receive the instruction for the task; and
a drive unit configured to move the autonomous moving apparatus according to the task.

9. A management method for managing an autonomous moving apparatus, the management method comprising:
setting a cleanliness level of the autonomous moving apparatus;
receiving an instruction for a task executed by the autonomous moving apparatus;
storing a database, the database associating information about at least one of an operator of the autonomous moving apparatus, a destination of the autonomous moving apparatus, a place which the autonomous moving apparatus passes through, and an object to be conveyed by the autonomous moving apparatus, which are included in the task, with the cleanliness level of the autonomous moving apparatus; and
controlling the cleanliness level of the autonomous moving apparatus or processing for the instruction based on the information stored in the database.

10. A program for causing a computer to execute a management method for managing an autonomous moving apparatus, the method comprising:
setting a cleanliness level of the autonomous moving apparatus;
receiving an instruction for a task executed by the autonomous moving apparatus;
storing a database, the database associating information about at least one of an operator of the autonomous moving apparatus, a destination of the autonomous moving apparatus, a place which the autonomous moving apparatus passes through, and an object to be conveyed by the autonomous moving apparatus, which are included in the task, with the cleanliness level of the autonomous moving apparatus; and
controlling the cleanliness level of the autonomous moving apparatus or processing for the instruction based on the information stored in the database.
